# EUROPEAN PATENT APPLICATION

(11) **EP 4 506 361 A1**
(43) Date of publication of application: **12.02.2025**
(21) Application number: 23778442.6
(22) Date of filing: 31.03.2023
(51) Int. Cl.: C07K 16/28, C12N 15/13, A61K 39/395, A61P 35/00

(54) **ANTIBODY AGAINST C-MET AND USE THEREOF**

(30) Priority: 02.04.2022 CN 202210345737
(71) Applicant: Biotheus Inc., Tangjiawan Town, Xiangzhou District Zhuhai, Guangdong 519080 (CN)
(72) Inventor: WU, Fan, Zhuhai, Guangdong 519080 (CN); MIAO, Xiaoniu, Zhuhai, Guangdong 519080 (CN); LUO, Yi, Zhuhai, Guangdong 519080 (CN); WANG, Ping, Zhuhai, Guangdong 519080 (CN); ZHAO, Zhenting, Zhuhai, Guangdong 519080 (CN)
(74) Representative: Cabinet Nony
(86) International application number: PCT/CN2023/085417
(87) International publication number: WO 2023/186078

(57) **Abstract**

The present application relates to an antibody capable of specifically binding to c-Met or antigen-binding fragment thereof, and an immunoconjugate, a pharmaceutical composition and a kit comprising the same. The present application also relates to use of the antibody or antigen-binding fragment thereof in the preparation of a kit or a drug. The antibody has a relatively high affinity for cells expressing or overexpressing c-Met, and also has some thermal stability. Moreover, the antibody can inhibit the HGF-c-Met signal pathway, thereby causing cancer cells to undergo apoptosis and thus inhibiting the proliferation of cancer cells. The antibody has great potential for application in targeted therapy for tumors.

## Description

### Technical Field

The present application belongs to the field of biomedical technology. More specifically, the present application relates to an antibody or antigen-binding fragment thereof capable of specifically binding to c-Met, as well as an immunoconjugate, pharmaceutical composition and kit containing the antibody or antigen-binding fragment thereof. The present application also relates to the use of the antibody or antigen-binding fragment thereof in the preparation of a kit or medicament.

### Background

The c-Met protein is a receptor tyrosine kinase, which is converted from a 170kDa leader protein into an α subunit of 50kDa and a β subunit of 145kDa through post-translational modification. It forms a transmembrane dimer after disulfide bond connection. At present, the main known ligand of c-Met is hepatocyte growth factor (HGF). Upon receipt of HGF stimulation, the intracellular domain of c-Met undergoes auto-phosphorylation at tyrosine residues Y1234 and Y1235, and then the phosphorylation signal is transduced to Y1349 and Y1356, enabling the intracellular domain of c-Met bind to the adaptor proteins. The downstream signal activation pathways of c-Met include PI3K/Akt, Rac1/Cdc42, and Erk/MAPK, which can significantly affect relevant manifestations such as cell proliferation, migration, infiltration, and tube tissue formation. After the c-Met protein is activated by autophosphorylation, the Cb1 ubiquitin ligase initiates the ubiquitination of this protein, then enters the degradation process, and performs negative regulation on the c-Met pathways.

Studies have shown that c-Met can promote cancer progression and is highly expressed in a variety of tumor tissues. This makes it a target for the development of anticancer drugs. In addition to traditional small molecule RTK inhibitors, the drug development of large molecule targeting antibodies has gradually advanced and deepened into clinical research. The example thereof includes the anti-c-Met monoclonal antibody MetMab. Other examples are ADC molecules such as TR1801, which is conjugated with the antitubulin toxin tesirine, is effective in mouse tumor PDX models with low, medium, and high abundance expression of MET.

Therefore, it is necessary to develop a new anti-c-Met monoclonal antibody and its corresponding humanized antibody to improve affinity and facilitate production.

### Contents of the Invention

After a lot of experiments and repeated exploration, the inventors of the present application have provided an antibody or antigen-binding fragment thereof capable of specifically binding to c-Met. The antibody has a high affinity with cells that express or overexpress c-Met and has a certain thermal stability. In addition, the antibody can inhibit the HGF-c-Met signaling pathway, thereby causing apoptosis of cancer cells and inhibiting the proliferation of cancer cells.

Therefore, in the first aspect, the present application provides an antibody or antigen-binding fragment thereof capable of specifically binding to c-Met, wherein the antibody or antigen-binding fragment thereof comprises:
(a) a heavy chain variable region (VH) comprising the following 3 complementary determining regions (CDRs):
   (i) VH CDR1, which is composed of a sequence as set forth in any one of SEQ ID NOs: 19, 21, 23, 25, 27, 29, 31, 33 or 35, or a sequence having a substitution, deletion or addition of one or several amino acids (e.g., a substitution, deletion or addition of 1, 2 or 3 amino acids) as compared thereto,
   (ii) VH CDR2, which is composed of a sequence as set forth in any one of SEQ ID NOs: 37, 39, 41, 43, 45, 47, 49, 51 or 53, or a sequence having a substitution, deletion or addition of one or several amino acids (e.g., a substitution, deletion or addition of 1, 2 or 3 amino acids) as compared thereto, and
   (iii) VH CDR3, which is composed of a sequence as set forth in any one of SEQ ID NOs: 55, 57, 59, 61, 63, 65, 67, 69 or 71, or a sequence having a substitution, deletion or addition of one or several amino acids (e.g., a substitution, deletion or addition of 1, 2 or 3 amino acids) as compared thereto;
   and/or,
(b) a light chain variable region (VL) comprising the following 3 complementary determining regions (CDRs):
   (iv) VL CDR1, which is composed of a sequence as set forth in any one of SEQ ID NOs: 20, 22, 24, 26, 28, 30, 32, 34 or 36, or a sequence having a substitution, deletion or addition of one or several amino acids (e.g., a substitution, deletion or addition of 1, 2 or 3 amino acids) as compared thereto,
   (v) VL CDR2, which is composed of a sequence as set forth in any one of SEQ ID NOs: 38, 40, 42, 44, 46, 48, 50, 52 or 54, or a sequence having a substitution, deletion or addition of one or several amino acids (e.g., a substitution, deletion or addition of 1, 2 or 3 amino acids) as compared thereto, and
   (vi) VL CDR3, which is composed of a sequence as set forth in any one of SEQ ID NOs: 56, 58, 60, 62, 64, 66, 68, 70 or 72, or a sequence having a substitution, deletion or addition of one or several amino acids (e.g., a substitution, deletion or addition of 1, 2 or 3 amino acids) as compared thereto.

In certain embodiments, the substitution described in any one of (i) to (vi) is a conservative substitution.

In certain embodiments, the CDR as described in any one of (i) to (vi) is defined according to the Kabat, IMGT or Chothia numbering system.

In certain embodiments, the CDR as described in any one of (i) to (vi) is defined according to the IMGT numbering system.

In certain embodiments, the antibody or antigen-binding fragment thereof as described above comprises: the following 3 heavy chain CDRs: VH CDR1 as set forth in SEQ ID NO: 19, VH CDR2 as set forth in SEQ ID NO: 37, VH CDR3 as set forth in SEQ ID NO: 55; and/or, the following 3 light chain CDRs: VL CDR1 as set forth in SEQ ID NO: 20, VL CDR2 as set forth in SEQ ID NO: 38, VL CDR3 as set forth in SEQ ID NO: 56.

In certain embodiments, the antibody or antigen-binding fragment thereof as described above comprises: the following 3 heavy chain CDRs: VH CDR1 as set forth in SEQ ID NO: 21, VH CDR2 as set forth in SEQ ID NO: 39, VH CDR3 as set forth in SEQ ID NO: 57; and/or, the following 3 light chain CDRs: VL CDR1 as set forth in SEQ ID NO: 22, VL CDR2 as set forth in SEQ ID NO: 40, VL CDR3 as set forth in SEQ ID NO: 58.

In certain embodiments, the antibody or antigen-binding fragment thereof as described above comprises: the following 3 heavy chain CDRs: VH CDR1 as set forth in SEQ ID NO: 23, VH CDR2 as set forth in SEQ ID NO: 41, VH CDR3 as set forth in SEQ ID NO: 59; and/or, the following 3 light chain CDRs: VL CDR1 as set forth in SEQ ID NO: 24, VL CDR2 as set forth in SEQ ID NO: 42, VL CDR3 as set forth in SEQ ID NO: 60.

In certain embodiments, the antibody or antigen-binding fragment thereof as described above comprises: the following 3 heavy chain CDRs: VH CDR1 as set forth in SEQ ID NO: 25, VH CDR2 as set forth in SEQ ID NO: 43, VH CDR3 as set forth in SEQ ID NO: 61; and/or, the following 3 light chain CDRs: VL CDR1 as set forth in SEQ ID NO: 26, VL CDR2 as set forth in SEQ ID NO: 44, VL CDR3 as set forth in SEQ ID NO: 62.

In certain embodiments, the antibody or antigen-binding fragment thereof as described above comprises: VH CDR1 as set forth in SEQ ID NO: 27, VH CDR2 as set forth in SEQ ID NO: 45, VH CDR3 as set forth in SEQ ID NO: 63; and/or, the following 3 light chain CDRs: VL CDR1 as set forth in SEQ ID NO: 28, VL CDR2 as set forth in SEQ ID NO: 46, VL CDR3 as set forth in SEQ ID NO: 64.

In certain embodiments, the antibody or antigen-binding fragment thereof as described above comprises: the following 3 heavy chain CDRs: VH CDR1 as set forth in SEQ ID NO: 29, VH CDR2 as set forth in SEQ ID NO: 47, VH CDR3 as set forth in SEQ ID NO: 65; and/or, the following 3 light chain CDRs: VL CDR1 as set forth in SEQ ID NO: 30, VL CDR2 as set forth in SEQ ID NO: 48, VL CDR3 as set forth in SEQ ID NO: 66.

In certain embodiments, the antibody or antigen-binding fragment thereof as described above comprises: the following 3 heavy chain CDRs: VH CDR1 as set forth in SEQ ID NO: 31, VH CDR2 as set forth in SEQ ID NO: 49, VH CDR3 as set forth in SEQ ID NO: 67; and/or, the following 3 light chain CDRs: VL CDR1 as set forth in SEQ ID NO: 32, VL CDR2 as set forth in SEQ ID NO: 50, VL CDR3 as set forth in SEQ ID NO: 68.

In certain embodiments, the antibody or antigen-binding fragment thereof as described above comprises: the following 3 heavy chain CDRs: VH CDR1 as set forth in SEQ ID NO: 33, VH CDR2 as set forth in SEQ ID NO: 51, VH CDR3 as set forth in SEQ ID NO: 69; and/or, the following 3 light chain CDRs: VL CDR1 as set forth in SEQ ID NO: 34, VL CDR2 as set forth in SEQ ID NO: 52, VL CDR3 as set forth in SEQ ID NO: 70.

In certain embodiments, the antibody or antigen-binding fragment thereof as described above comprises: the following 3 heavy chain CDRs: VH CDR1 as set forth in SEQ ID NO: 35, VH CDR2 as set forth in SEQ ID NO: 53, VH CDR3 as set forth in SEQ ID NO: 71; and/or, the following 3 light chain CDRs: VL CDR1 as set forth in SEQ ID NO: 36, VL CDR2 as set forth in SEQ ID NO: 54, VL CDR3 as set forth in SEQ ID NO: 72.

In certain embodiments, the antibody or antigen-binding fragment thereof further comprises a framework region of a human immunoglobulin.

In certain embodiments, the antibody or antigen-binding fragment thereof as described above comprises:
(a) a heavy chain variable region (VH) comprising an amino acid sequence selected from the following:
   (i) the sequence as set forth in any one of SEQ ID NOs: 1, 3, 5, 7, 9, 11, 13, 15, or 17;
   (ii) a sequence having a substitution, deletion or addition of one or several amino acids (e.g., a substitution, deletion or addition of 1, 2, 3, 4, or 5 amino acids) as compared to the sequence as set forth in any one of SEQ ID NOs: 1, 3, 5, 7, 9, 11, 13, 15, or 17; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 1, 3, 5, 7, 9, 11, 13, 15, or 17;
   and/or
(b) a light chain variable region (VL) comprising an amino acid sequence selected from the group consisting of:
   (iv) the sequence as set forth in SEQ ID NO: 2, 4, 6, 8, 10, 12, 14, 16 or 18;
   (v) a sequence having a substitution, deletion or addition of one or several amino acids (e.g., a substitution, deletion or addition of 1, 2, 3, 4, or 5 amino acids) as compared to a sequence as set forth in SEQ ID NO: 2, 4, 6, 8, 10, 12, 14, 16 or 18; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2, 4, 6, 8, 10, 12, 14, 16 or 18.

In certain embodiments, the substitution described in (ii) or (v) is a conservative substitution.

In certain embodiments, the above-mentioned antibody or antigen-binding fragment thereof comprises: a VH having a sequence as set forth in SEQ ID NO: 1 and a VL having a sequence as set forth in SEQ ID NO: 2.

In certain embodiments, the above-mentioned antibody or antigen-binding fragment thereof comprises: a VH having a sequence as set forth in SEQ ID NO: 3 and a VL having a sequence as set forth in SEQ ID NO: 4.

In certain embodiments, the above-mentioned antibody or antigen-binding fragment thereof comprises: a VH having a sequence as set forth in SEQ ID NO: 5 and a VL having a sequence as set forth in SEQ ID NO: 6.

In certain embodiments, the above-mentioned antibody or antigen-binding fragment thereof comprises: a VH having a sequence as set forth in SEQ ID NO: 7 and a VL having a sequence as set forth in SEQ ID NO: 8.

In certain embodiments, the above-mentioned antibody or antigen-binding fragment thereof comprises: a VH having a sequence as set forth in SEQ ID NO: 9 and a VL having a sequence as set forth in SEQ ID NO: 10.

In certain embodiments, the above-mentioned antibody or antigen-binding fragment thereof comprises: a VH having a sequence as set forth in SEQ ID NO: 11 and a VL having a sequence as set forth in SEQ ID NO: 12.

In certain embodiments, the above-mentioned antibody or antigen-binding fragment thereof comprises: a VH having a sequence as set forth in SEQ ID NO: 13 and a VL having a sequence as set forth in SEQ ID NO: 14.

In certain embodiments, the above-mentioned antibody or antigen-binding fragment thereof comprises: a VH having a sequence as set forth in SEQ ID NO: 15 and a VL having a sequence as set forth in SEQ ID NO: 16.

In certain embodiments, the above-mentioned antibody or antigen-binding fragment thereof comprises: a VH having a sequence as set forth in SEQ ID NO: 17 and a VL having a sequence as set forth in SEQ ID NO:18.

In certain embodiments, the antibody or antigen-binding fragment thereof comprises:
(a) a heavy chain constant region (CH) of a human immunoglobulin or variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived; and/or
(b) a light chain constant region (CL) of a human immunoglobulin or variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived.

In certain embodiments, the heavy chain constant region is an IgG heavy chain constant region, such as an IgG1, IgG2, IgG3 or IgG4 heavy chain constant region.

In certain embodiments, the light chain constant region is a κ light chain constant region or a λ light chain constant region.

In certain embodiments, the heavy chain constant region has a sequence as set forth in SEQ ID NO: 121.

In certain embodiments, the light chain constant region has a sequence as set forth in SEQ ID NO: 122.

In certain embodiments, in the antibody or antigen-binding fragment thereof as described above, the antigen-binding fragment is selected from the group consisting of Fab, Fab', (Fab')², Fv, disulfide-linked Fv, scFv, diabody and single domain antibody (sdAb).

In certain embodiments, the antibody is a murine antibody, a chimeric antibody, a humanized antibody, or a multispecific antibody.

In certain embodiments, the antigen-binding fragment is an Fab.

In certain embodiments, the antigen-binding fragment further comprises an Fc fragment (e.g., an Fc fragment of human IgG1) or mutant thereof.

In certain embodiments, the Fc fragment has a LALA mutation and a knob mutation; or, the Fc fragment has a LALA mutation and a hole mutation.

In certain embodiments, the Fc fragment has a sequence as set forth in SEQ ID NO: 111 or 112.

In another aspect, the present application provides an isolated nucleic acid molecule, which encodes the antibody or antigen-binding fragment thereof as described above.

In another aspect, the present application provides a vector, which comprises the nucleic acid molecule as described above. In certain embodiments, the vector is a cloning vector or an expression vector.

In another aspect, the present application provides a host cell, which comprises the nucleic acid molecule as described above or the vector as described above. In certain embodiments, the host cell is a mammalian cell.

In another aspect, the present application provides a method for preparing the antibody or antigen-binding fragment thereof as described above, comprisesing culturing the host cell as described above under a condition that allows expression of the antibody or antigen-binding fragment thereof, and recovering the antibody or antigen-binding fragment thereof from a culture of the cultured host cell.

In another aspect, the present application provides a multispecific molecule, which comprises the antibody or antigen-binding fragment thereof as described above.

In certain embodiments, the multispecific molecule is capable of specifically binding to c-Met and additionally specifically binding to one or more other targets.

In certain embodiments, the multispecific molecule is a bispecific molecule.

In certain embodiments, the bispecific molecule further comprises a molecule (e.g., a second antibody) having a second binding specificity for a second target.

In another aspect, the present application provides an immunoconjugate, which comprises the antibody or antigen-binding fragment thereof as described above or the multispecific molecule as described above, and a therapeutic agent linked to the antibody or antigen-binding fragment thereof or the multispecific molecule.

In certain embodiments, the therapeutic agent is selected from cytotoxic agents.

In certain embodiments, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof.

In certain embodiments, the immunoconjugate is an antibody-drug conjugate (ADC).

In another aspect, the present application provides a pharmaceutical composition, which comprises the antibody or antigen-binding fragment thereof as described above, or the multispecific molecule as described above, or the immunoconjugate as described above, and a pharmaceutically acceptable carrier and/or excipient.

In certain embodiments, the pharmaceutical composition further comprises an additional pharmaceutically active agent.

In certain embodiments, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the antibody or antigen-binding fragment thereof, multispecific molecule or immunoconjugate and the additional pharmaceutically active agent are provided as separate components or as components of the same composition.

In another aspect, the present application provides a kit, which comprises the antibody or antigen-binding fragment thereof as described above.

In certain embodiments, the antibody or antigen-binding fragment thereof comprises a detectable label, such as an enzyme (e.g., horseradish peroxidase), a radionuclide, a fluorescent dye, a luminescent substance (e.g., a chemiluminescent substance), or biotin.

In certain embodiments, the kit further comprises a second antibody capable of specifically recognizing the antibody or antigen-binding fragment thereof as described above.

In certain embodiments, the second antibody further comprises a detectable label, such as an enzyme (e.g., horseradish peroxidase), a radionuclide, a fluorescent dye, a luminescent substance (e.g., a chemiluminescent substance), or biotin.

In another aspect, the present application provides a chimeric antigen receptor, which comprises an antigen binding domain of the antibody or antigen binding fragment thereof as described above.

In certain embodiments, the antigen binding domain comprises a heavy chain variable region and a light chain variable region of the antibody or antigen binding fragment thereof as described above.

In certain embodiments, the antigen binding domain is a scFv.

In certain embodiments, the chimeric antigen receptor is expressed by an immune effector cell (e.g., a T cell).

In another aspect, the present application provides a method for inhibiting the growth of tumor cells expressing c-Met and/or killing the tumor cells, comprising contacting the tumor cells with an effective amount of the antibody or antigen binding fragment thereof as described above, or the multispecific molecule as described above, or the immunoconjugate as described above, or the pharmaceutical composition as described above, or the chimeric antigen receptor as described above.

In another aspect, the present application provides a use of the antibody or antigen-binding fragment thereof as described above, or the multispecific molecule as described above, or the immunoconjugate as described above, or the pharmaceutical composition as described above, or the chimeric antigen receptor as described above, in the manufacture of a medicament for preventing and/or treating a tumor in a subject (e.g., a human).

In certain embodiments, the medicament further comprises an additional pharmaceutically active agent.

In certain embodiments, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the tumor expresses c-Met.

In certain embodiments, the tumor involves a tumor cell expressing c-Met. In certain embodiments, the c-Met is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In some embodiments, the subject is a mammal, such as a human.

The use of the antibody or antigen-binding fragment thereof as described above in the manufacture of a kit for determining whether a tumor can be treated by an anti-tumor therapy targeting c-Met.
(1) contacting a sample containing the tumor cell with the antibody or antigen-binding fragment thereof as described above;
(2) detecting the formation of a complex comprising the antibody or antigen-binding fragment thereof and c-Met.

In some embodiments, the antibody or antigen-binding fragment thereof comprises a detectable label.

In some embodiments, the c-Met is c-Met of a mammal (e.g., a human, a monkey).

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In another aspect, the present application provides a method for preventing and/or treating a tumor in a subject, the method comprising administering to the subject in need thereof an effective amount of the antibody or antigen-binding fragment thereof as described above, or the bispecific or multispecific molecule as described above, or the immunoconjugate as described above, or the pharmaceutical composition as described above, or the chimeric antigen receptor as described above, or the host cell as described above.

In certain embodiments, the tumor expresses c-Met.

In certain embodiments, the tumor involves a tumor cell expressing c-Met. In certain embodiments, the c-Met is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In certain embodiments, the subject is a mammal, such as a human.

In certain embodiments, the method further comprises administering an additional drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus.

In certain embodiments, the method further comprises administering an additional anti-tumor therapy, such as a surgery, a chemotherapy, a radiotherapy, a targeted therapy, an immunotherapy, a hormone therapy, a gene therapy or a palliative therapy.

In another aspect, the present application provides a method for determining whether a tumor can be treated by an anti-tumor therapy targeting c-Met, comprising the following steps:
(1) contacting a sample containing the tumor cell with the antibody or antigen-binding fragment thereof as described above;
(2) detecting the formation of a complex comprising the antibody or antigen-binding fragment thereof and c-Met.

In certain embodiments, the antibody or antigen-binding fragment thereof comprises a detectable label.

In certain embodiments, the c-Met is c-Met of a mammalian (e.g., a human, a monkey).

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In another aspect, the present application provides a method for determining the presence or amount of c-Met in a sample, which comprises the following steps:
(1) contacting the sample with the antibody or antigen-binding fragment thereof as described above;
(2) detecting the formation of a complex comprising the antibody or antigen-binding fragment thereof and c-Met or detecting the amount of the complex.

In certain embodiments, the antibody or antigen-binding fragment thereof comprises a detectable label.

In certain embodiments, the c-Met is c-Met of a mammal (e.g., a human, a monkey).

### Definition of terms

In the present invention, unless otherwise specified, the scientific and technical terms used herein have the meanings commonly understood by those skilled in the art. In addition, the operation steps of molecular genetics, nucleic acid chemistry, chemistry, molecular biology, biochemistry, cell culture, microbiology, cell biology, genomics and recombinant DNA used herein are all conventional steps widely used in the corresponding fields. At the same time, in order to better understand the present invention, the definitions and explanations of relevant terms are provided below.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of the classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminus to the carboxyl terminus in the following sequence: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia or IMGT numbering systems.

As used herein, the term "framework region" or "FR" residues refers to those amino acid residues in the variable region of an antibody other than the CDR residues defined above.

The term "antibody" is not limited to any particular method of producing the antibody. For example, it includes recombinant antibody, monoclonal antibody and polyclonal antibody. The antibody can be an antibody of different isotypes, for example, IgG (e.g., IgG1, IgG2, IgG3 or IgG4 subtype), IgA1, IgA2, IgD, IgE or IgM antibody.

As used herein, the terms "monoclonal antibody", "McAb", "mAb" have the same meaning and are used interchangeably, and refer to an antibody or a fragment of an antibody derived from a group of highly homologous antibody molecules, that is, a group of identical antibody molecules except for natural mutations that may occur spontaneously. Monoclonal antibody has high specificity for a single epitope on an antigen. Polyclonal antibody is mentioned relatively to monoclonal antibody, which usually contains at least 2 or more different antibodies, which usually recognize different epitopes on an antigen. In addition, the modifier "monoclonal" only indicates that the antibody is characterized by being obtained from a highly homologous antibody group, and it should not be interpreted that the antibody needs to be prepared by any specific method.

The monoclonal antibody of the present invention can be prepared by a variety of techniques, such as hybridoma technology (see, for example, Kohler et al., Nature, 256:495, 1975), recombinant DNA technology (see, for example, U.S. Patent Application 4,816,567), or phage antibody library technology (see, for example, Clackson et al. Nature 352: 624-628, 1991, or Marks et al. J. Mol. Biol. 222: 581-597, 1991).

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')², Fd, Fv, complementarity determining region (CDR) fragment, scFv, diabody, single domain antibody, chimeric antibody, linear antibody, nanobody (technology from Domantis), probody, and such polypeptides, which contain at least a portion of an antibody that is sufficient to confer specificity to the polypeptides with antigen-binding capability. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and disulfide bonds between the HRs of the two full-length heavy chains. The full-length antibody of the present invention may be from a single species, such as human; and it may also be a chimeric antibody or humanized antibody. The full-length antibody of the present invention comprises two antigen binding sites formed by VH and VL pairs, respectively, which specifically recognize/bind to the same antigen.

As used herein, the term "Fd" refers to an antibody fragment consisting of VH and CH1 domains; the term "dAb fragment" refers to an antibody fragment consisting of VH domain (Ward et al., Nature 341:544 546 (1989)); the term "Fab fragment" refers to an antibody fragment consisting of VL, VH, CL and CH1 domains; the term "F(ab')² fragment" refers to an antibody fragment comprising two Fab fragments linked by a disulfide bridge at the hinge region; the term "Fab' fragment" refers to a fragment obtained by reducing the disulfide bond linking the two heavy chain fragments in the F(ab')² fragment, which consists of a complete light chain and an Fd fragment of heavy chain (consisting of VH and CH1 domains).

As used herein, the term "Fv" refers to an antibody fragment consisting of VL and VH domains of a single arm of an antibody. The Fv fragment is generally considered to be the smallest antibody fragment that can form a complete antigen binding site. It is generally believed that the six CDRs confer antigen binding specificity to an antibody. However, even a single variable region (e.g., a Fd fragment, which contains only three CDRs specific for an antigen) can recognize and bind to an antigen, although its affinity may be lower than that of the complete binding site.

As used herein, the term "Fc" refers to an antibody fragment formed by disulfide bonding between the second and third constant regions of the first heavy chain of an antibody and the second and third constant regions of the second heavy chain. The Fc fragment of an antibody has a variety of different functions but does not participate in antigen binding.

As used herein, the term "scFv" refers to a single polypeptide chain comprising VL and VH domains, wherein the VL and VH are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, ed., Springer-Verlag, New York, pp. 269-315 (1994)). Such scFv molecule may have a general structure: NH2-VL-linker-VH-COOH or NH2-VH-linker-VL-COOH. A suitable prior art linker may consist of repeated GGGGS amino acid sequence or variant thereof. For example, a linker having the amino acid sequence (GGGGS)4 may be used, but variants thereof may also be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, there may also be a disulfide bond between the VH and VL of the scFv. In certain embodiments of the present invention, the scFv may form a di-scFv, which refers to an antibody formed by connecting two or more single scFvs in series. In certain embodiments of the present invention, scFv can form (scFv)2, which refers to an antibody formed by two or more single scFvs in parallel.

As used herein, the term "single-domain antibody (sdAb)" has the meaning generally understood by those skilled in the art, which refers to an antibody fragment composed of a single monomer variable antibody domain (e.g., a single heavy chain variable region), which retains the ability to specifically bind to the same antigen bound by a full-length antibody. Single-domain antibody is also called nanobody.

Each of the above antibody fragments retains the ability to specifically bind to the same antigen bound by a full-length antibody, and/or competes with the full-length antibody for specific binding to the antigen.

Antigen-binding fragments of an antibody (e.g., the above antibody fragments) can be obtained from a given antibody (e.g., the antibody provided by the present invention) using conventional techniques known to those skilled in the art (e.g., recombinant DNA technology or enzymatic or chemical cleavage methods), and the antigen-binding fragments of the antibody can be screened for specificity in the same manner as for intact antibody.

As used herein, unless the context clearly indicates otherwise, when the term "antibody" is referred to, it includes not only intact antibody, but also antigen-binding fragments of the antibody.

As used herein, the term "chimeric antibody" refers to an antibody in which a portion of the light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but in any case, it still retains binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include an antibody in which the heavy chain and light chain variable regions of the antibody are derived from a first antibody, and the heavy chain and light chain constant regions of the antibody are derived from a second antibody.

As used herein, the term "identity" is used to refer to the matching of sequences between two polypeptides or between two nucleic acids. To determine the percent identity of two amino acid sequences or two nucleic acid sequences, the sequences are aligned for optimal comparison purposes (e.g., a gap may be introduced in a first amino acid sequence or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences (i.e., percent identity = number of identical overlapping positions/total number of positions × 100%). In certain embodiments, the two sequences are of the same length.

The determination of the percent identity between two sequences can also be accomplished using a mathematical algorithm. A non-limiting example of a mathematical algorithm for the comparison of two sequences is the algorithm of Karlin and Altschul, 1990, Proc. Natl. Acad. Sci. U.S.A. 87:2264-2268, as modified by Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403.

As used herein, the term "variant", in the context of polypeptide (including a polypeptide), also refers to a polypeptide or peptide comprising an amino acid sequence that has been altered by the introduction of substitution, deletion or addition of amino acid residues. In some cases, the term "variant" also refers to a polypeptide or peptide that has been modified (i.e., by covalently attaching any type of molecule to the polypeptide or peptide). For example, but not limiting, the polypeptide can be modified, for example, by glycosylation, acetylation, pegylation, phosphorylation, amidation, derivatization by a known protecting/blocking group, proteolytic cleavage, attachment to cellular ligand or other protein, etc. Derivatized polypeptides or peptides can be produced by chemical modification using techniques known to those skilled in the art, including but not limited to specific chemical cleavage, acetylation, formylation, metabolic synthesis of tunicamycin, etc. In addition, the variant has similar, identical or improved functions to the polypeptide or peptide from which it is derived.

As used herein, the term "specific binding" refers to a non-random binding reaction between two molecules, such as a reaction between an antibody and an antigen to which it is directed. The strength or affinity of a specific binding interaction can be expressed by the equilibrium dissociation constant (KD) of the interaction. In the present invention, the term "KD" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

The specific binding properties between two molecules can be determined using methods known in the art. One method involves measuring the rate of formation and dissociation of antigen binding sites/antigen complexes. Both the "association rate constant" (ka or kon) and the "dissociation rate constant" (kdis or koff) can be calculated from the concentrations and the actual rates of association and dissociation (see, Malmqvist M, Nature, 1993, 361: 186-187). The ratio of kdis/kon is equal to the dissociation constant KD (see, Davies et al., Annual Rev Biochem, 1990; 59: 439-473). The values of KD, kon and kdis can be measured by any effective method. In certain embodiments, the dissociation constant can be measured in Biacore using surface plasmon resonance (SPR). In addition, the dissociation constant can be measured by bioluminescence interferometry or Kinexa.

As used herein, the detectable label of the present invention can be any substance that can be detected by fluorescence, spectroscopy, photochemistry, biochemistry, immunology, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances, such as acridinium ester compounds, luminol and its derivatives, ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid vehicle into which a polynucleotide can be inserted. When a vector can express the protein encoded by the inserted polynucleotide, the vector is called an expression vector. The vector can be introduced into a host cell by transformation, transduction or transfection, so that the genetic material elements it carries are expressed in the host cell. Vectors are well known to those skilled in the art, and include but are not limited to: plasmids; phagemids; cosmids; artificial chromosomes, such as yeast artificial chromosome (YAC), bacterial artificial chromosome (BAC) or P1-derived artificial chromosome (PAC); bacteriophages such as λ phage or M13 phage and animal viruses. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription start sequence, enhancer sequence, selection element and reporter gene. In addition, the vector may also contain a replication start site.

As used herein, the term "a host cell" refers to a cell that can be used to introduce a vector, including but not limited to prokaryotic cells such as *Escherichia coli* or *Bacillus subtilis,* fungal cells such as yeast cells or *Aspergillus,* insect cells such as *S2 Drosophila* cells or Sf9, or animal cells such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, but not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), a solution containing a surfactant (e.g., 0.01% polysorbate 20), a pH buffer solution (e.g., a phosphate buffer solution), Ringer's solution, and any combination thereof.

As used herein, the term "prevention" refers to a method implemented to prevent or delay the occurrence of a disease, disorder, or symptom in a subject. As used herein, the term "treatment" refers to a method implemented to obtain a beneficial or desired clinical result. For the purposes of the present invention, beneficial or desired clinical results include (but are not limited to) alleviation of symptoms, reduction of the extent of the disease, stabilization (i.e., no longer worsening) of the state of the disease, delay or slowing the progression of the disease, improvement or alleviation of the state of the disease, and relief of symptoms (whether partial or complete), whether detectable or undetectable. In addition, "treatment" can also refer to prolonging survival compared to the expected survival (if not receiving treatment).

As used herein, the term "subject" refers to a mammal, such as a human, a cynomolgus monkey, or a mouse. In certain embodiments, the subject (e.g., a human, a cynomolgus monkey, or a mouse) suffers from a disease associated with c-Met, or is at risk of suffering from the above-mentioned disease.

As used herein, the term "effective amount" refers to an amount sufficient to obtain or at least partially obtain the desired effect. For example, a disease-preventing effective amount refers to an amount sufficient to prevent, arrest, or delay the occurrence of the disease; a disease-treating effective amount refers to an amount sufficient to cure or at least partially prevent the disease and its complications in a patient already suffering from the disease. Determining such an effective amount is well within the capabilities of those skilled in the art. For example, the amount effective for therapeutic use will depend on the severity of the disease to be treated, the overall state of the patient's own immune system, the patient's general condition such as age, weight and gender, the mode of administration of drug, and other treatments administered at the same time, etc.

As used herein, the term "single-armed antibody" refers to an antigen-binding fragment comprising a Fab segment and an Fc segment, generally the Fab segment comprises a heavy chain (e.g., VH and CH1) and a light chain (e.g., VL and CL), and the Fc segment comprises a constant region (e.g., CH2 and CH3). The Fab segment and the Fc segment may be connected by a linker or not. The single-armed antibody of the present invention can be prepared or synthesized by a variety of methods, for example, constructing the sequence encoding the Fab heavy chain and the sequence encoding the Fc into the same vector, and constructing the sequence encoding the Fab light chain into another vector, and transforming the two vectors into a host cell separately to obtain a single-armed antibody.

As used herein, the term "bispecific antibody" refers to a conjugate formed by a first antibody (or a fragment thereof) and a second antibody (or a fragment thereof) or an antibody analog through a coupling arm, and the coupling method includes but is not limited to chemical reaction, gene fusion and enzymatic. Bispecific antibodies can be linked or generated by various methods, for example, see the method of Songsivilai et al. (Clin. Exp. Immunol., 79: 315-321 (1990)), and the method of Kostelny et al. (J. Immunol., 148: 1547-1553 (1992)).

### Beneficial effects of the invention

Compared with the prior art, the antibody or antigen-binding fragment thereof prepared in the present application can specifically bind to the c-Met, and then specifically recognize or bind to cells expressing c-Met. The antibody has a high affinity with cells expressing or overexpressing c-Met and has a certain thermal stability. In addition, the antibody can inhibit the HGF-c-Met signaling pathway, thereby causing apoptosis of cancer cells and inhibiting the proliferation of cancer cells, and has great application potential in targeted tumor therapy.

The embodiments of the present invention will be described in detail below in conjunction with the drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows a schematic diagram of the structure of the antibody constructed in Example 2 of the present application.
Fig. 2 shows the binding activity of the anti-c-Met antibodies of the present application to cells overexpressing c-Met; wherein, Fig. 2A and Fig. 2B show the binding activity of the antibodies (antibodies 22, 23, 74, 111, 136, 187, 216, 221, 223) of the present application and the control antibodies to HEK293-Hu c-Met cells; and Fig. 2C and Fig. 2D show the binding activity of the antibodies (antibodies 22, 23, 74, 111, 136, 187, 216, 221, 223) of the present application and the control antibodies to HEK293-Rhe c-Met cells.
Fig. 3 shows the inhibition activity of the anti-c-Met antibodies (antibodies 22, 23, 74, 111, 136, 187, 216, 221, 223) of the present application and the control antibodies on HCC827 cells.
Fig. 4 shows the inhibition of the anti-c-Met antibodies (antibodies 136, 187) of the present application and the control antibodies on H596 cells.

### Sequence information

The information of some sequences involved in the present invention is provided in Table 1 below.

**Table 1: Description of sequences**

| SEQ ID NO: | Description | Sequence |
|---|---|---|
| 1 | 22-VH | QVQLVQSGAEVKKPGASVKVSCKTSGYTFTNYGISWVRQAPGQG LEWMGWISAYNGHTNYAQKLQGRVTMTTDTSTSTAYMELRSLRS DDTAVYYCARDRRTGTSFFDYWGQGTLVTVSS |
| 2 | 22-VL | DIVMTQSPSSVSASVGDRVTITCRASQGISSWLAWYQQKPGKAPKL LIYAASSLQSGVPSKFSGSGSGTEFTLTISSLQPEDFATYFCQQAYGF PLTFGGGTKVEIK |
| 3 | 23-VH | EVQLVESGGGLVQPGGSLRLSCAASGFTFSSYAMTWVRQAPGKGL EWVSVISGSGGTTYYADSVKGRFTISRDNSKNTLYLQMHSLRAED TAVYYCAKVITFERGRTFDIWGQGTMVTVSS |
| 4 | 23-VL | DIVMTQSPSSLSASVGDRVTITCRASQSFSDYLNWYRQKPGKAPKL LLFAASSLQSGVPSRFSGSGSGTDFTLTISSLQPEDFATYYCQQSYSP PYTFGQGTKLEIK |
| 5 | 74-VH | QLQLQESGPGLVKPSETLSLTCTVSGGSISSSSYYWGWIRQSPGKGL EWIGSIYYSGNTYYNPSLKSRVTISVDTSKNQFSLKLSSVTAADTAV YYCVRQVYDFWRDWGQGTLVTVSS |
| 6 | 74-VL | DIVMTQSPSSVSASVGDRVTITCRASQGINSWLAWYQQKPGKAPN LLIYAASSLQSGVPSRFSGRGSGTDFSLTISSLHPEDFATYYCQQAK GFPLTFGGGTKVEIK |
| 7 | 111-VH | QVQLVQSGAEVKKPGASMKVSCKASGNTFISYGINWVRQAPGQG LEWMGWISAYNGNTNYAQKFQGRVTMTTDTSTSSAYMELRNLRS DDTAVYYCATGDTTSSGYYNYYMDVWGKGTTVTVSS |
| 8 | 111-VL | EIVLTQSPGTLSLSPGERATLSCRASQSVSSSYLAWYQQKPGQAPRL LIYGAFSRATGIPDRFSGSGSGTDFTLTISRLEPEDFAVYYCQQYGSS PRTFGQGTKVDIK |
| 9 | 136-VH | QVQLQESGPGLVKPSETLSLTCTVSGGSVTSVNYYWKWIRQPPGK GLEWIGYISYSGNTNYNPSLKSRVTISVDTSKNQFSLKLSSVTAADT AVYYCVRAPYYYMDVWSKGTTVTVSS |
| 10 | 136-VL | DIVMTQSPSSVSASVGDRVTITCRASQGISSWLAWYQQKPGKAPKL LIYAVSSLQSGVPSRFSGSGSGTDFTLTISSLQPEDFATYFCQQANSF PLTFGGGTKVEIK |
| 11 | 187-VH | QLQLQESGPGLVKPSETLSLTCTVSGGSISSSSYYWGWIRQSPGKGL EWIGSRYYSGNTYYNPSLKSRVTMSVDTSKNQFSLKLRSVTAADT AVYYCARQVYDYWRDWGQGALVTVSS |
| 12 | 187-VL | DIVMTQSPSSVSASVGDRVTITCRASQGISSWLAWYQQKPGKAPNL LIYAASNLPSGVPSRFSGSGSGTVFTLTISSLQPEDFATYYCQQSNSF PLTFGGGTKVEIK |
| 13 | 216-VH | EVQLVESGGGLVQPGGSLRLFCGASGFTFSSYAMSWVRQAPGKGL EWVSAITGSGGSTYYADSVKGRFTISRDNSKNTLFLQLNSLRAEDT AIYYCAKIVTVEAGRWLDPWGQGTMVTVSS |
| 14 | 216-VL | DIRMTQSPSSLSASVGDRVTITCRASQSISNYLNWFQQKPGKAPKLL IYATSSLQSGVPSRFSGSGSGTDFTLTISSLQPEDFATYYCQQSYSIP YTFGQGTKVEIK |
| 15 | 221-VH | EVQLLESGGGLVQPGGSLRLSCAASGFTFSSYAMTWVRQAPGKGL EWVSVISGSGGSTYYEDSVKGRFTISRDNSKNTLYLQMNSLRAEDT AVYYCAKVITFERGRTFDIWGQGTMVTVSS |
| 16 | 221-VL | EIVLTQSPGTLSLSPGERATLSCRASQSVSSSYLAWYQQKPGQAPRL LIYGPSSRATGIPDRFSGSGSGTDFTLTISSLQPADFASYYCQQSYITP YTFGQGTKVEIK |
| 17 | 223-VH | EVQLVESGGGLVQPGGSLRLSCAASGFTFRNYAMSWVRQAPGKG LEWVSAISGSGVGTYYADSVKGRFTISRDNSKNTLFLQMNSLRVED TAVYYCVRVMTVEFSRWLDPWGQGTLVTVSS |
| 18 | 223-VL | DIQLTQSPSSLSASVGDRVTITCRASQSIITYLNWYQQKPGKAPKLLI YAASSLQSGVPSRFSGSGSGTDFTLNISSLQPEDFATYYCQQANSLP YTFGQGTKVEIK |
| 19 | 22-VH CDR1 | GYTFTNYG |
| 20 | 22-VL CDR1 | QGISSW |
| 21 | 23-VH CDR1 | GFTFSSYA |
| 22 | 23-VL CDR1 | QSFSDY |
| 23 | 74-VH CDR1 | GGSISSSSYY |
| 24 | 74-VL CDR1 | QGINSW |
| 25 | 111-VH CDR1 | GNTFISYG |
| 26 | 111-VL CDR1 | QSVSSSY |
| 27 | 136-VH CDR1 | GGSVTSVNYY |
| 28 | 136-VL CDR1 | QGISSW |
| 29 | 187-VH CDR1 | GGSISSSSYY |
| 30 | 187-VL CDR1 | QGISSW |
| 31 | 216-VH CDR1 | GFTFSSYA |
| 32 | 216-VL CDR1 | QSISNY |
| 33 | 221-VH CDR1 | GFTFSSYA |
| 34 | 221-VL CDR1 | QSVSSSY |
| 35 | 223-VH CDR1 | GFTFRNYA |
| 36 | 223-VL CDR1 | QSIITY |
| 37 | 22-VH CDR2 | ISAYNGHT |
| 38 | 22-VL CDR2 | AAS |
| 39 | 23-VH CDR2 | ISGSGGTT |
| 40 | 23-VL CDR2 | AAS |
| 41 | 74-VH CDR2 | IYYSGNT |
| 42 | 74-VL CDR2 | AAS |
| 43 | 111-VH CDR2 | ISAYNGNT |
| 44 | 111-VL CDR2 | GAF |
| 45 | 136-VH CDR2 | ISYSGNT |
| 46 | 136-VL CDR2 | AVS |
| 47 | 187-VH CDR2 | RYYSGNT |
| 48 | 187-VL CDR2 | AAS |
| 49 | 216-VH CDR2 | ITGSGGST |
| 50 | 216-VL CDR2 | ATS |
| 51 | 221-VH CDR2 | ISGSGGST |
| 52 | 221-VL CDR2 | GPS |
| 53 | 223-VH CDR2 | ISGSGVGT |
| 54 | 223-VL CDR2 | AAS |
| 55 | 22-VH CDR3 | ARDRRTGTSFFDY |
| 56 | 22-VL CDR3 | QQAYGFPLT |
| 57 | 23-VH CDR3 | AKVITFERGRTFDI |
| 58 | 23-VL CDR3 | QQSYSPPYT |
| 59 | 74-VH CDR3 | VRQVYDFWRD |
| 60 | 74-VL CDR3 | QQAKGFPLT |
| 61 | 111-VH CDR3 | ATGDTTSSGYYNYYMDV |
| 62 | 111-VL CDR3 | QQYGSSPRT |
| 63 | 136-VH CDR3 | VRAPYYYMDV |
| 64 | 136-VL CDR3 | QQANSFPLT |
| 65 | 187-VH CDR3 | ARQVYDYWRD |
| 66 | 187-VL CDR3 | QQSNSFPLT |
| 67 | 216-VH CDR3 | AKIVTVEAGRWLDP |
| 68 | 216-VL CDR3 | QQSYSIPYT |
| 69 | 221-VH CDR3 | AKVITFERGRTFDI |
| 70 | 221-VL CDR3 | QQSYITPYT |
| 71 | 223-VH CDR3 | VRVMTVEFSRWLDP |
| 72 | 223-VL CDR3 | QQANSLPYT |
| 73 | Heavy chain upstream primer 1 | ACAGGTGCCCACTCCCAGGTGCAG |
| 74 | Heavy chain upstream primer 2 | AAGGTGTCCAGTGTGARGTGCAG |
| 75 | Heavy chain upstream primer 3 | CCCAGATGGGTCCTGTCCCAGGTGCAG |
| 76 | Heavy chain upstream primer 4 | CAAGGAGTCTGTTCCGAGGTGCAG |
| 77 | Heavy chain downstream primer 1 | TGAGCAGCAGAACCTGGAGCCAAGGGATAGACAGATGGGGCTG TTGT |
| 78 | Heavy chain downstream primer 2 | TGAGCAGCAGAACCTGGAGCCAGTGGATAGACAGATGGGGCTG TTGT |
| 79 | Light chain upstream primer 1 | ATGAGGSTCCCYGCTCAGCTGCTGG |
| 80 | Light chain upstream primer 2 | CTCTTCCTCCTGCTACTCTGGCTCCCAG |
| 81 | Light chain upstream primer 3 | ATTTCTCTGTTGCTCTGGATCTCTG |
| 82 | Light chain downstream primer 1 | TGTTCAGAAGATGGTGGGAAGATAGATACAGTTGGTGCAGCAT CAGC |
| 83 | Heavy chain upstream primer 5 | GAGAAGCTGTTGCTGAAGCTTCTTTGGACAAGAGACAGGTGCA GCTGGTGCAG |
| 84 | Heavy chain upstream primer 6 | GAGAAGCTGTTGCTGAAGCTTCTTTGGACAAGAGAGAGGTGCA GCTGGTGCAG |
| 85 | Heavy chain upstream primer 7 | GAGAAGCTGTTGCTGAAGCTTCTTTGGACAAGAGAGAGGTGCA GCTGGTGGAG |
| 86 | Heavy chain upstream primer 8 | GAGAAGCTGTTGCTGAAGCTTCTTTGGACAAGAGAGAGGTGCA GCTGTTGGAG |
| 87 | Heavy chain upstream primer 9 | GAGAAGCTGTTGCTGAAGCTTCTTTGGACAAGAGACAGGTGCA GCTGCAGGAG |
| 88 | Heavy chain upstream primer 10 | GAGAAGCTGTTGCTGAAGCTTCTTTGGACAAGAGACAGGTGCA GCTACAGCAGTG |
| 89 | Heavy chain upstream primer 11 | GAGAAGCTGTTGCTGAAGCTTCTTTGGACAAGAGACAGGTTCAG CTGGTGCAG |
| 90 | Heavy chain upstream primer 12 | GAGAAGCTGTTGCTGAAGCTTCTTTGGACAAGAGACAGGTCCA GCTGGTACAG |
| 91 | Heavy chain upstream primer 13 | GAGAAGCTGTTGCTGAAGCTTCTTTGGACAAGAGACAGGTGCA GCTGGTGGAG |
| 92 | Heavy chain upstream primer 14 | GAGAAGCTGTTGCTGAAGCTTCTTTGGACAAGAGAGAAGTGCA GCTGGTGGAG |
| 93 | Heavy chain upstream primer 15 | GAGAAGCTGTTGCTGAAGCTTCTTTGGACAAGAGACAGCTGCA GCTGCAGGAG |
| 94 | Heavy chain upstream primer 16 | GAGAAGCTGTTGCTGAAGCTTCTTTGGACAAGAGACAGGTACA GCTGCAGCAG |
| 95 | Heavy chain downstream primer 3 | GCCAGGGGGAAGACCGATGGGCCCTTGGTGCTAGCTGAGGAGA CGGTGACCAG |
| 96 | Heavy chain downstream primer 4 | GCCAGGGGGAAGACCGATGGGCCCTTGGTGCTAGCTGAAGAGA CGGTGACCATTG |
| 97 | Heavy chain downstream primer 5 | GCCAGGGGAAGACCGATGGGCCCTTGGTGCTAGCTGAGGAGA CGGTGACCGTG |
| 98 | Light chain upstream primer 4 | CCTTCTTTTTCTCTTTAGCTCGGCTTATTCCGACATCCAGATGAC CCAGTC |
| 99 | Light chain upstream primer 5 | CCTTCTTTTTCTCTTTAGCTCGGCTTATTCCGACATCCAGTTGAC CCAGTCT |
| 100 | Light chain upstream primer 6 | CCTTCTTTTTCTCTTTAGCTCGGCTTATTCCGATATCCGGATGAC CCAGTC |
| 101 | Light chain upstream primer 7 | CCTTCTTTTTCTCTTTAGCTCGGCTTATTCCGATATTGTGATGAC CCAGAC |
| 102 | Light chain upstream primer 8 | CCTTCTTTTTCTCTTTAGCTCGGCTTATTCCGATATTGTGATGACT CAGTC |
| 103 | Light chain upstream primer 9 | CCTTCTTTTTCTCTTTAGCTCGGCTTATTCCGATGTTGTGATGACT CAGTC |
| 104 | Light chain upstream primer 10 | CCTTCTTTTTCTCTTTAGCTCGGCTTATTCCGAAATTGTGTTGAC ACAGTC |
| 105 | Light chain upstream primer 11 | CCTTCTTTTTCTCTTTAGCTCGGCTTATTCCGAAATAGTGATGAC GCAGTC |
| 106 | Light chain upstream primer 12 | CCTTCTTTTTCTCTTTAGCTCGGCTTATTCCGAAATTGTGTTGAC GCAGTCT |
| 107 | Light chain upstream primer 13 | CCTTCTTTTTCTCTTTAGCTCGGCTTATTCCGACATCGTGATGAC CCAGTC |
| 108 | Light chain downstream primer 2 | GGAAGATGAAGACAGATGGTGCAGCCACCGTACGTTTGATYTC CACCTTGGTC |
| 109 | Light chain downstream primer 3 | GGAAGATGAAGACAGATGGTGCAGCCACCGTACGTTTGATCTC CAGCTTGGTC |
| 110 | Light chain downstream primer 4 | GGAAGATGAAGACAGATGGTGCAGCCACCGTACGTTTGATATC CACTTTGGTC |
| 111 | Human IgG1-Fc (LALA mutation, knob mutation) | DKTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPCRDE LTKNQVSLWCLVKGFYPSDIAVEWESNGQPENNYKTTPPVLDSDG SFFLYSKLTVDKSRWQQGNVFSCSVMHEALHNHYTQKSLSLSPG |
| 112 | Fc-LALA-hole | DKTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVCTLPPSRDE |
| | | LTKNQVSLSCAVKGFYPSDIAVEWESNGQPENNYKTTPPVLDSDGS FFLVSKLTVDKSRWQQGNVFSCSVMHEALHNHYTQKSLSLSPG |
| 113 | Knob heavy chain of single-arm antibody Amivantamab | QVQLVQSGAEVKKPGASVKVSCETSGYTFTSYGISWVRQAPGHGL EWMGWISAYNGYTNYAQKLQGRVTMTTDTSTSTAYMELRSLRSD DTAVYYCARDLRGTNYFDYWGQGTLVTVSSASTKGPSVFPLAPSS KSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGGPSVFLFPPKPKDTLMISRTPEVTCVVVDVSHED PEVKFNWYVDGVEVHNAKTKPREEQYNSTYRVVSVLTVLHQDWL NGKEYKCKVSNKALPAPIEKTISKAKGQPREPQVYTLPPCRDELTK NQVSLWCLVKGFYPSDIAVEWESNGQPENNYKTTPPVLDSDGSFF LYSKLTVDKSRWQQGNVFSCSVMHEALHNHYTQKSLSLSPG |
| 114 | Light chain of single-arm antibody Amivantamab | DIQMTQSPSSVSASVGDRVTITCRASQGISNWLAWFQHKPGKAPKL LIYAASSLLSGVPSRFSGSGSGTDFTLTISSLQPEDFATYYCQQANSF PITFGQGTRLEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC |
| 115 | Hole heavy chain of single-arm antibody Amivantamab and antibody onartuzumab | DKTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVCTLPPSRDE LTKNQVSLSCAVKGFYPSDIAVEWESNGQPENNYKTTPPVLDSDGS FFLVSKLTVDKSRWQQGNVFSCSVMHEALHNHYTQKSLSLSPG |
| 116 | Konb heavy chain of single-arm antibody LY2875358 | QVQLVQSGAEVKKPGASVKVSCKASGYTFTDYYMHWVRQAPGQ GLEWMGRVNPNRRGTTYNQKFEGRVTMTTDTSTSTAYMELRSLR SDDTAVYYCARANWLDYWGQGTTVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPCRDELTKN QVSLWCLVKGFYPSDIAVEWESNGQPENNYKTTPPVLDSDGSFFL YSKLTVDKSRWQQGNVFSCSVMHEALHNHYTQKSLSLSPG |
| 117 | Light chain of single-arm antibody LY2875358 | DIQMTQSPSSLSASVGDRVTITCSVSSSVSSIYLHWYQQKPGKAPKL LIYSTSNLASGVPSRFSGSGSGTDFTLTISSLQPEDFATYYCQVYSGY PLTFGGGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 118 | Hole heavy chain of single-arm antibody LY2875358 | DKTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVCTLPPSRDE LTKNQVSLSCAVKGFYPSDIAVEWESNGQPENNYKTTPPVLDSDGS FFLVSKLTVDKSRWQQGNVFSCSVMHEALHNHYTQKSLSLSPG |
| 119 | Knob heavy chain of antibody onartuzumab | EVQLVESGGGLVQPGGSLRLSCAASGYTFTSYWLHWVRQAPGKG LEWVGMIDPSNSDTRFNPNFKDRFTISADTSKNTAYLQMNSLRAED TAVYYCATYRSYVTPLDYWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPCRDELTKN QVSLWCLVKGFYPSDIAVEWESNGQPENNYKTTPPVLDSDGSFFL YSKLTVDKSRWQQGNVFSCSVMHEALHNHYTQKSLSLSPG |
| 120 | Light chain of antibody onartuzumab | DIQMTQSPSSLSASVGDRVTITCKSSQSLLYTSSQKNYLAWYQQKP GKAPKLLIYWASTRESGVPSRFSGSGSGTDFTLTISSLQPEDFATYY CQQYYAYPWTFGQGTKVEIKRTVAAPSVFIFPPSDEQLKSGTASVV CLLNNFYPREAKVQWKVDNALQSGNSQESVTEQDSKDSTYSLSST LTLSKADYEKHKVYACEVTHQGLSSPVTKSFNRGEC |
| 121 | CH1 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSC |
| 122 | CL | |

### Specific Models for Carrying Out the Invention

The present invention is now described with reference to the following examples which are intended to illustrate the present invention (but not to limit the present invention).

Unless otherwise specified, the experiments and methods described in the examples are basically carried out according to conventional methods well known in the art and described in various references. For example, conventional techniques of immunology, biochemistry, chemistry, molecular biology, microbiology, cell biology, genomics and recombinant DNA used in the present invention can be found in Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al., eds., (1987)); METHODS IN ENZYMOLOGY series (Academic Publishing Company): PCR 2: A PRACTICAL METHOD. APPROACH) (M.J. MacPherson, B.D. Hames and G.R. Taylor, ed. (1995)), and ANIMAL CELL CULTURE (R.I. Freshney, ed. (1987)).

In addition, if the specific conditions were not specified in the examples, they were carried out according to conventional conditions or conditions recommended by the manufacturer. The reagents or instruments used without indicating the manufacturer were all conventional products that could be obtained commercially. It is known to those skilled in the art that the examples describe the present invention by way of example and are not intended to limit the scope of protection of the present invention. All the disclosures and other references mentioned herein are incorporated herein by reference in their entirety.

### Example 1: screening of anti-c-Met antibody

### 1.1. Construction of antibody library

### Animal immunization

1 mg of cellular mesenchymal epithelial transition factor (c-Met) antigen (purchased from AcroBiosystems) was mixed with an equal volume of Freund's adjuvant, and used to immunize 5 humanized mice (purchased from Alloy Company), once a week for a total of 4 immunizations, to stimulate B cells to produce antigen-specific antibodies. After the 4 immunizations, the spleens of the mice were harvested and total RNA was extracted using an RNA extraction reagent Trizol (purchased from Invitrogen). The cDNA of fully humanized mice was obtained by reverse transcription using a cDNA synthesis kit (purchased from Invitrogen).

### Antibody gene amplification

First round of PCR: Using heavy chain upstream primers 1 to 4 (SEQ ID NO: 73 to SEQ ID NO: 76) and heavy chain downstream primers 1 to 2 (SEQ ID NO: 77 and SEQ ID NO: 78), as well as light chain upstream primers 1 to 3 (SEQ ID NO: 79 to SEQ ID NO: 81) and light chain downstream primer 1 (SEQ ID NO: 82), as shown in Table 1, the sequences of the heavy and light chain variable regions of the fully humanized antibody were amplified from the cDNA.

The above PCR products were subjected to agarose gel electrophoresis to determine the band size and uniformity. The target fragments were at 400bp to 500bp.

Second round of PCR: Using heavy chain upstream primers 5 to 16 (SEQ ID NO: 83 to SEQ ID NO: 94) and heavy chain downstream primers 3 to 5 (SEQ ID NO: 95 to SEQ ID NO: 97), as well as light chain upstream primers 4 to 13 (SEQ ID NO: 98 to SEQ ID NO: 107) and light chain downstream primers 2 to 4 (SEQ ID NO: 108 to SEQ ID NO: 110), as shown in Table 1, the products of the first round of PCR were used as templates for the second round of sequence amplification to add homology arms to the heavy and light chain genes.

The target fragments were recovered using a PCR purification kit (purchased from QIAGEN).

### Library construction

The linearized yeast display vector and the product of the second round of PCR were mixed and electrotransformed into *Saccharomyces cerevisiae* (purchased from ATCC) to construct a fully humanized anti-c-Met antibody library from five mice, and the library capacity was determined. The library capacity was 3×10⁷.

### 1.2. Screening of c-Met antibodies

### Biotinylation labeling of c-Met protein

An appropriate volume of double distilled water was taken to dissolve human c-Met protein (purchased from AcroBiosystems). According to the instructions of the biotin labeling kit (purchased from Thermo), the biotin was dissolved and mixed with the protein solution, then incubated at 4°C for 2 hours. A desalting column (purchased from Thermo) was used to remove excess biotin, in which the desalting column pretreatment and sample collection operations were all carried out according to the steps in the product instructions.

### Magnetic bead sorting (MACS) to enrich yeast capable of specifically binding to human c-Met

The antibody library constructed in Example 1.1 was inoculated into SD-CAA amplification medium (1L of SD-CAA amplification medium contained 6.7g of YNB, 5g of tyrosine, 13.62g of Na₂HPC₄·12H₂O, 7.44g of NaH₂PO₄ and 2% glucose), and cultured overnight at 30°C and 225rpm. An appropriate amount of yeast cells was taken, centrifuged at 3000rpm×5min (the following centrifugation operations were the same) to remove the culture medium, and then the yeast cells were resuspended with SD-CAA induction medium, and induced overnight. The concentration of the library after induction was determined, and an appropriate amount of yeast cells was harvested, and centrifuged to remove the culture medium. The yeast cells were resuspended with 50ml of PBS, and centrifuged to remove the supernatant. The yeast cells were resuspended with 10ml of PBS.

The biotin-labeled human c-Met protein (its final concentration was 100nM) was added, incubated at room temperature for 30min, and the yeast cells were collected by centrifugation, and washed 3 times with 50ml of PBS. The yeast cells were resuspended with 5ml of washing solution, added with 200µl of SA magnetic beads (purchased from Miltenyi Biotec), The samples were incubated for 10 minutes. The mixture of yeast and magnetic beads was washed 3 times with PBS, and the mixture was added to LS purification column (purchased from Miltenyi Biotec). The LS purification column was placed on a magnetic stand and washed with PBS to remove non-specifically bound yeast cells. The purification column was taken from the magnetic stand and added with PBS to elute the yeast cells. The eluted yeast cells were centrifuged and transferred to SD-CAA amplification medium for amplification.

### Flow cytometry sorting (FACS) to obtain high-affinity yeast cells

The yeast cells enriched by MACS were inoculated into SD-CAA amplification medium, and cultured in a shake flask overnight at 30°C and 225rpm. The yeast cells were resuspended in SD-CAA induction medium and induced overnight. Anti-c-Myc mouse antibody (purchased from Thermo) and 100nM biotin-labeled c-Met antigen were added and incubated for 10min. The yeast cells were washed 3 times with PBS, added with goat anti-mouse IgG (H+L) Alexa Fluor Plus 488 fluorescent antibody (purchased from Invitrogen) and streptavidin APC conjugate fluorescent antibody (purchased from Invitrogen), and incubated for 15min. The cells were resuspended in PBS and sorted using a BD AriaIII instrument to obtain yeast with high binding ability to the c-Met antigen.

### Retrieval of c-Met antibody candidate molecule antibody gene

The yeast solution with high binding ability to human c-Met antigen obtained by MACS and FACS enrichment was coated on a solid culture plate of SD-CAA, and then single clones were picked and cultured overnight at 30°C and 225rpm in SD-CAA amplification medium. The amplified single clone was treated with 0.1% SDS, centrifuged and the supernatant was used as a template for PCR amplification. The PCR product was subjected to sequencing to obtain the gene sequence. A total of 9 antibodies were obtained, which were named antibodies 22, 23, 74, 111, 136, 187, 216, 221 and 223, and their specific sequences were shown in Table 1, among which the CDR sequences of the antibodies were determined by the IMGT numbering system (Lefranc et al., Dev. Comparat. Immunol. 27:55-77, 2003).

### Example 2: Construction and expression and purification of single-arm antibodies

### 2.1. Constructing antibody gene into pCDNA3.1 expression vector

The nucleotide sequence encoding the heavy chain variable region (as set forth in SEQ ID NO: 1, 3, 5, 7, 9, 11, 13, 15 or 17) was ligated to the nucleotide sequence encoding the heavy chain constant region 1 (as set forth in SEQ ID NO: 121) and the nucleotide sequence encoding the human IgG1-Fc (LALA mutation, knob mutation) segment (SEQ ID NO: 111), and cloned into a linearized pCDNA3.1 vector that had been double-digested with EcoRI and NotI restriction enzymes(purchased from Vazyme), using a homologous recombination system; the nucleotide sequence encoding the light chain variable region (as set forth in SEQ ID NO: 2, 4, 6, 8, 10, 12, 14, 16 or 18) and the nucleotide sequence encoding the light chain constant region (as set forth in SEQ ID NO: 122) were cloned into an *EcoR* I/*Xhol* I double-digestion linearized pCDNA3.1 vector; the nucleotide sequence encoding Fc-LALA-hole (SEQ ID NO: 112) was cloned into an *EcoR* I/*Xhol* I double-digestion linearized pCDNA3.1 vector. The process was carried out in accordance with the product manual. The homologous recombination product was transformed into Top10 competent cells, plated on an ampicillin-resistant plate, then cultured at 37°C overnight, single colonies were picked for sequencing, and plasmids were extracted.

### 2.2. Cell transfection and protein purification

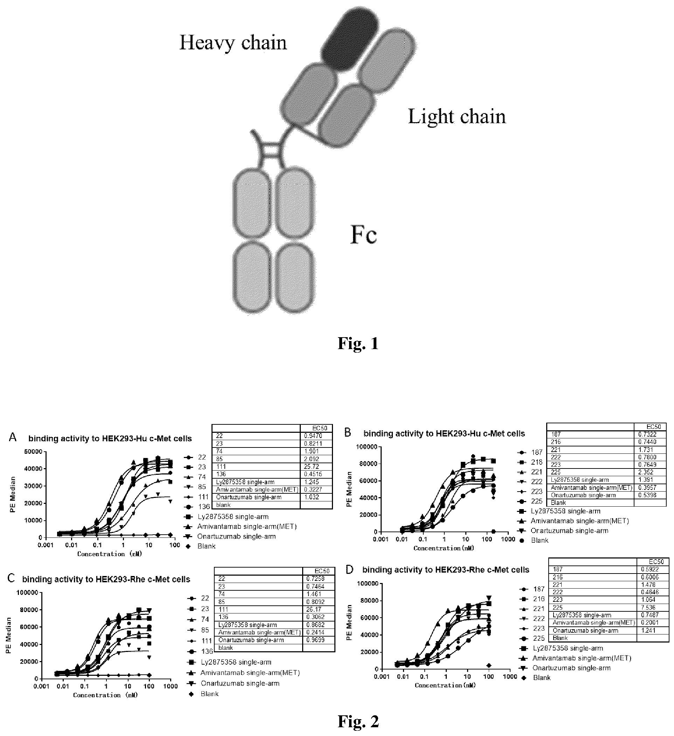

ExpiCHO^{™} expression system kit (purchased from Thermo) was used to co-transfect the above-extracted heavy chain (Fc-LALA-knob), light chain and Fc-LALA-hole (SEQ ID NO: 112) plasmids into Expi-CHO cells to produce antibodies with a single Fab structure (Fig. 1). The transfection method was carried out in accordance with the product manual. After 5 days of cell culture, the supernatant was collected and the target protein was purified by protein A magnetic beads (purchased from GenScript) sorting method. The magnetic beads were resuspended in an appropriate volume of binding buffer (PBS + 0.1% Tween 20, pH 7.4) (1 to 4 times the volume of the magnetic beads), added to the sample to be purified, and incubated at room temperature for 1 hour with gentle shaking. The sample was placed on a magnetic stand (purchased from Beaver), the supernatant was discarded, and the magnetic beads were washed 3 times with binding buffer. Elution buffer (0.1M sodium citrate, pH3.2) was added according to 3 to 5 times the volume of the magnetic beads, shaken at room temperature for 5 to 10 minutes, and placed back on the magnetic stand, and the elution buffer was collected and transferred to a collection tube with neutralization buffer (1M Tris, pH 8.54) and mixed well to obtain the target protein.

### Example 3: Determination of c-Met antibody affinity

ForteBio affinity determination was performed according to existing methods (Estep, P et al., Determination of antibody-antigen affinity and epitope binding based on high-throughput methods. MAbs, 2013.5(2): p.270-8). Briefly, the sensor was equilibrated offline in assay buffer for 30 min, then testing was performed online for 60 s to establish a baseline, and the purified antibody obtained as described above was loaded online onto the AHQ sensor. The sensor was then placed in 100 nM human c-Met antigen for 5 min, and then transferred to PBS for dissociation for 5 min. The kinetic analysis was performed using a 1:1 binding model.

**Table 2: Affinity of candidate molecules with human c-Met**

| No. | KD(M) | Kon(1/Ms) | Koff(1/s) |
|---|---|---|---|
| 22 | 4.29E-09 | 1.28E+05 | 5.48E-04 |
| 23 | 5.96E-10 | 1.60E+05 | 9.50E-05 |
| 74 | 6.01E-09 | 1.79E+05 | 1.08E-03 |
| 111 | 2.34E-09 | 1.60E+05 | 3.74E-04 |
| 136 | 2.93E-11 | 2.29E+05 | 6.71E-06 |
| 187 | 1.68E-09 | 2.20E+05 | 3.69E-04 |
| 216 | 6.54E-10 | 2.19E+05 | 1.43E-04 |
| 221 | 2.01E-09 | 1.84E+05 | 3.70E-04 |
| 223 | 9.76E-09 | 1.67E+05 | 1.63E-03 |

The candidate molecules were also tested for cross-reactivity with proteins from different species, including proteins from cynomolgus monkey (purchased from AcroBiosystems).

**Table 3: Affinity of candidate molecules with monkey c-Met**

| No. | KD(M) | Kon(1/Ms) | Koff(1/s) |
|---|---|---|---|
| 22 | 1.90E-08 | 1.36E+05 | 2.59E-03 |
| 23 | 2.65E-08 | 5.69E+04 | 1.51E-03 |
| 74 | 9.70E-09 | 2.69E+05 | 2.60E-03 |
| 111 | 1.15E-08 | 1.02E+05 | 1.17E-03 |
| 136 | 1.16E-09 | 3.69E+05 | 4.26E-04 |
| 187 | 2.92E-09 | 3.24E+05 | 9.47E-04 |
| 216 | 3.84E-09 | 1.28E+05 | 4.93E-04 |
| 221 | 6.38E-08 | 3.01E+04 | 1.92E-03 |
| 223 | 1.62E-08 | 6.73E+04 | 1.09E-03 |

The experimental results were shown in Tables 2 and 3. The 9 antibodies of the present application all had a certain affinity with the c-Met proteins of mammals (e.g., humans, monkeys).

The construction, expression and purification method of protein was the same as in Example 2.2, and the purity of the protein was obtained by HPLC detection. The HPLC method was as follows, mobile phase: 150mM Na₂HPO₄•12H₂O, pH7.0; chromatographic conditions: detection wavelength: 280 nm, column temperature: 25°C, flow rate: 0.35 ml/min, detection time: 20 min, Zenix-C SEC-300 chromatographic column (SEPAX 4.6×300mm, 3µm).

**Table 4: Purity detection results of anti-c-Met antibodies**

| No. | Monomer ratio (%) |
|---|---|
| 22 | 97.20% |
| 23 | 98.50% |
| 74 | 98.00% |
| 111 | 97.20% |
| 136 | 94.80% |
| 187 | 97.70% |
| 216 | 97.30% |
| 221 | 94.00% |
| 223 | 96.80% |

The experimental results were shown in Table 4. All of the 9 antibodies in the present application had a relatively high purity, that was, all at 94% and above.

### Example 4. Thermal stability of anti-c-Met candidates

DSC (Differential scanning calorimetry) was used to detect the thermal stability of different antibodies. The sample was concentrated and diluted with PBS to 1 mg/ml; 5000× fluorescent colorimetric agent Cypro Orange (purchased from Bio-Rad) was diluted 50 times with ultrapure water to obtain 100× fluorescent colorimetric agent Sypro Orange. 50µl of 1mg/ml sample was taken, added with 10µl of the 100× fluorescent colorimetric agent Sypro Orange and 40µl of ultrapure water, mixed well, 30µl of the mixture was taken and added to a 96-well PCR plate, 3 duplicate wells for each sample, and the plate was placed in a PCR instrument. The heating program was set as follows: keeping at a constant temperature of 25°C for 5min, and heating to 99°C at a rate of 0.5°C/min. After the program was completed, the temperature value of the lowest point of the curve in the "Melt Curve" graph was read, which was the Tm value of the sample. The specific results were shown in Table 5 below:

**Table 5: Tm values of anti-c-Met antibodies**

| No. | Tm (°C) |
|---|---|
| 22 | 67.34 |
| 23 | 67.95 |
| 74 | 67.42 |
| 111 | 64.26 |
| 136 | 67.86 |
| 187 | 67.42 |
| 216 | 67.42 |
| 221 | 67.42 |
| 223 | 67.42 |

The experimental results were shown in Table 5. All 9 antibodies of the present application had certain thermal stability.

### Example 5. Anti-c-Met purified candidates binding to human/rhesus monkey c-Met cells

In order to verify the binding ability of the antibodies of the present application to mammalian cells expressing c-Met, three existing antibodies Amivantamab, LY2875358 and onartuzumab were selected as control antibodies for comparison.

Among them, the construction process of Amivantamab and LY2875358 control antibodies was the same as that of the single-arm antibody of the present application, and both were constructed according to the steps of Example 2. In brief, for the control antibody Amivantamab, the insert fragments as set forth in SEQ ID NO:113, SEQ ID NO:114 and SEQ ID NO:115 were inserted into plasmids respectively, and the plasmids were co-transformed into Expi-CHO cells; for the control antibody LY2875358, the insert fragments as set forth in SEQ ID NO:116, SEQ ID NO:117 and SEQ ID NO:118 were inserted into plasmids respectively, and the plasmids were co-transformed into Expi-CHO cells. For the control antibody onartuzumab, the nucleotide sequences encoding SEQ ID NO:119, SEQ ID NO:120 and SEQ ID NO:115 were inserted into plasmids respectively, and the plasmids were co-transformed into Expi-CHO cells.

HEK293T cells overexpressing human c-Met (HEK293-Hu c-Met) and HEK293T cells overexpressing rhesus c-Met (HEK293-Rhe c-Met) were generated by transfecting the pCHO1.0 vector (purchased from Invitrogen) which human or rhesus c-Met cDNA (purchased from Sino Biological) had been cloned into the MCS. The cell density of the expanded HEK293-Hu c-Met/HEK293-Rhe c-Met cells was adjusted to 2×10⁶ cells/ml, added to a 96-well flow cytometry plate at 100 µl/well, and centrifuged for later use. The purified c-Met antibody was diluted with PBS, 3-fold dilution started from 100 nM for a total of 10 points. The diluted sample was added at 100 µl/well to the 96-well flow cytometry plate with cells, incubated at 4°C for 30 minutes, and washed twice with PBS. Goat F(ab')² Anti-Human IgG-Fc (PE) (purchased from Abcam) diluted 100 times with PBS was added at 100 µl/well, incubated at 4°C for 30 min, and washed twice with PBS. PBS was added at 100 µl/well to resuspend the cells, the cells were detected on a CytoFlex (Bechman) flow cytometer, and the corresponding MFI was calculated.

In the detection experiment of the above method, the experimental results were shown in Fig. 2A and Fig. 2B, and all anti-c-Met single-arm antibodies of the present invention had binding activity to HEK293-Hu c-Met cells. In particular, the antibody 136 showed strong binding activity similar to that of the control antibody Amivantamab, and was superior to the control antibody LY2875358 and the control antibody onartuzumab. Fig. 2C and Fig. 2D showed that the binding activity of all anti-c-Met single-arm antibodies of the present invention to HEK293-Rhe c-Met cells was similar to that to human c-Met overexpression cells. In particular, the antibody 136 showed strong binding activity similar to that of Amivantamab, and was superior to other candidate antibodies and the control antibodies. And as shown in Tables 6 and 7, all anti-c-Met screening single-arm antibodies showed similar binding ability (EC50) to cells expressing human c-Met and rhesus monkey c-Met.

**Table 6: Table for detecting binding activity of anti-c-Met single-arm antibodies to HEK293-Hu c-Met**

| Antibody clone No. | EC50 (nM) | EC50 (nM) |
|---|---|---|
| 22 | 0.947 | / |
| 23 | 0.8211 | / |
| 74 | 1.901 | / |
| 85 | 2.092 | / |
| 111 | 25.72 | / |
| 136 | 0.4515 | / |
| 187 | / | 0.7322 |
| 216 | / | 0.744 |
| 221 | / | 1.731 |
| 222 | / | 0.78 |
| 223 | / | 0.7649 |
| 225 | / | 2.352 |
| LY2875358 single-arm antibody | 1.245 | 1.391 |
| Amivantamab single-arm antibody | 0.3227 | 0.3957 |
| Onartuzumab | 1.032 | 0.5398 |

**Table 7: Detection of binding of anti-c-Met single-arm antibodies to HEK293-Rhe c-Met**

| Antibody clone No. | EC50 (nM) | EC50 (nM) |
|---|---|---|
| 22 | 0.7258 | / |
| 23 | 0.7464 | / |
| 74 | 1.461 | / |
| 85 | 0.8092 | / |
| 111 | 26.17 | / |
| 136 | 0.3062 | / |
| 187 | / | 0.5922 |
| 216 | / | 0.6006 |
| 221 | / | 1.478 |
| 222 | / | 0.4646 |
| 223 | / | 1.064 |
| 225 | / | 7.536 |
| LY2875358 single-arm antibody | 0.8682 | 0.7487 |
| Amivantamab single-arm antibody | 0.2414 | 0.2001 |
| Onartuzumab | 0.9699 | 1.241 |

### Example 6: Blocking HGF-dependent TKI resistance with single-arm antibodies for anti-c-Met screening

HCC827 cells were human non-small cell lung cancer cells and purchased from the Cell Bank of the Chinese Academy of Sciences, which could highly express epidermal growth factor receptor (EGFR) (exon 19 deletion) and c-Met receptor. The treatment with tyrosine kinase inhibitor (TKI) small molecule Gefitinib (gefitinib, an epidermal growth factor receptor tyrosine kinase inhibitor) could induce apoptosis in HCC827 cells. If HGF was added at the same time under such conditions, the c-Met pathway would be activated, thereby inducing HCC827 to resist to Gefitinib and inhibiting apoptosis.

The cell density of the expanded cultured HCC827 cells was adjusted to 2×10⁴ cells/ml, added at 100 µl/well to a 96-well cell culture plate, and cultured overnight for later use. Using 1640 medium, the antibody to be tested was diluted to 1000 nM with 1640 medium, HGF was diluted to 800 ng/mL, and Gefitinib was diluted to 8 µM. The diluted antibody at 50 µl/well, HGF at 25 µl/well, and Gefitinib at 25 µl/well were added to the 96-well plate with cells according to the experimental requirements, and the 1640 medium was supplemented to reach a total volume of 200 µl/well. After culturing for 3 days at 37°C and 5% carbon dioxide, 100 µl of the medium was removed, and then Cell titer glo (purchased from Promega) was added at 100 µl/well, and the chemiluminescent signal was collected with an microplate reader.

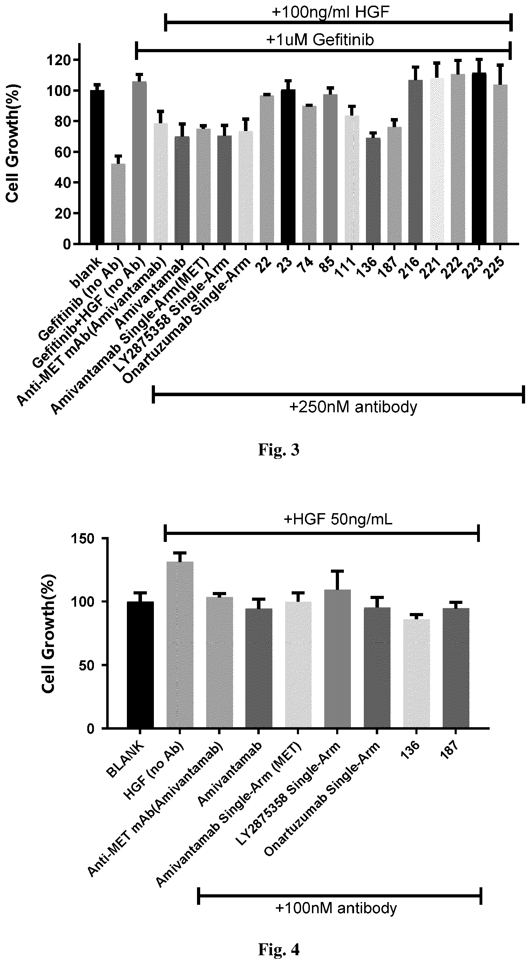

The experimental results were shown in Fig. 3. The single-arm antibodies for anti-c-Met screening of the present application inhibited the HGF-c-Met signaling pathway (e.g., antibodies 22, 23, 74, 85, 111, 136 and 187), thereby restoring the apoptosis of HCC827 induced by Gefitinib. Among them, the antibodies 136 and 187 both showed significant inhibitory activity, and were significantly better than other antibodies (e.g., antibodies 22, 23, 74.85, 111, 216, 221, 222, 223 and 225), and even better than some control antibodies.

### Example 7. Blocking HGF-induced cell proliferation with single-arm antibodies for anti-c-Met screening

H596 cells were human lung cancer cells and purchased from ATCC, which were capable of expressing EGFR and c-Met receptors. HGF treatment could induce H596 cell proliferation. If control antibodies were added under such conditions, the HGF-c-Met signaling pathway would be inhibited, thereby inhibiting HGF-induced proliferation of H596. The expanded H596 cells was adjusted to reach a density of 3×10⁴ cells/ml, added at 100 µl/well to a 96-well cell culture plate, and cultured overnight for later use. Using 1640 medium, the antibody to be tested was diluted to 400 nM, and HGF was diluted to 200 ng/mL. The diluted antibody at 50 µl/well, and HGF at 50 µl/well were added to the 96-well plate with cells according to the experimental requirements, and 1640 medium was supplemented to reach a total volume of 200 µl/well. The cells were cultured at 37 °C and 5% carbon dioxide for 5 days. After 5 days, 100 µl of the medium was removed, and then Cell titer glo (purchased from Promega) was added at 100 µl/well, and the chemiluminescent signal was collected with an microplate reader.

As shown in Fig. 4, the single-arm antibodies 136 and 187 for anti-c-Met screening both significantly inhibited HGF-induced the proliferation of H596 cells. In addition, the inhibitory effect of the antibody 136 was better than that of all control antibodies.

Although the specific embodiments of the present invention have been described in detail, those skilled in the art will understand that various modifications and changes can be made to the details based on all the teachings that have been disclosed, and these changes are within the scope of protection of the present invention. The entirety of the present invention is given by the appended claims and any equivalents thereof.

## Claims

1. An antibody or antigen-binding fragment thereof capable of specifically binding to c-Met, wherein the antibody or antigen-binding fragment thereof comprises:
(a) a heavy chain variable region (VH) comprising the following 3 complementary determining regions (CDRs):
(i) VH CDR1, which is composed of a sequence as set forth in any one of SEQ ID NOs: 19, 21, 23, 25, 27, 29, 31, 33 or 35, or a sequence having a substitution, deletion or addition of one or several amino acids (e.g., a substitution, deletion or addition of 1, 2 or 3 amino acids) as compared thereto,
(ii) VH CDR2, which is composed of a sequence as set forth in any one of SEQ ID NOs: 37, 39, 41, 43, 45, 47, 49, 51 or 53, or a sequence having a substitution, deletion or addition of one or several amino acids (e.g., a substitution, deletion or addition of 1, 2 or 3 amino acids) as compared thereto, and
(iii) VH CDR3, which is composed of a sequence as set forth in any one of SEQ ID NOs: 55, 57, 59, 61, 63, 65, 67, 69 or 71, or a sequence having a substitution, deletion or addition of one or several amino acids (e.g., a substitution, deletion or addition of 1, 2 or 3 amino acids) as compared thereto;
and/or,
(b) a light chain variable region (VL) comprising the following 3 complementary determining regions (CDRs):
(iv) VL CDR1, which is composed of a sequence as set forth in any one of SEQ ID NOs: 20, 22, 24, 26, 28, 30, 32, 34 or 36, or a sequence having a substitution, deletion or addition of one or several amino acids (e.g., a substitution, deletion or addition of 1, 2 or 3 amino acids) as compared thereto,
(v) VL CDR2, which is composed of a sequence as set forth in any one of SEQ ID NOs: 38, 40, 42, 44, 46, 48, 50, 52 or 54, or a sequence having a substitution, deletion or addition of one or several amino acids (e.g., a substitution, deletion or addition of 1, 2 or 3 amino acids) as compared thereto, and
(vi) VL CDR3, which is composed of a sequence as set forth in any one of SEQ ID NOs: 56, 58, 60, 62, 64, 66, 68, 70 or 72, or a sequence having a substitution, deletion or addition of one or several amino acids (e.g., a substitution, deletion or addition of 1, 2 or 3 amino acids) as compared thereto;
preferably, the substitution described in any one of (i) to (vi) is a conservative substitution;
preferably, the CDR described in any one of (i) to (vi) is defined according to the Kabat, IMGT or Chothia numbering system;
preferably, the CDR described in any one of (i) to (vi) is defined according to the IMGT numbering system.

2. The antibody or antigen-binding fragment thereof according to claim 1, comprising:
(1) the following 3 heavy chain CDRs: VH CDR1 as set forth in SEQ ID NO: 19, VH CDR2 as set forth in SEQ ID NO: 37, VH CDR3 as set forth in SEQ ID NO: 55; and/or, the following 3 light chain CDRs: VL CDR1 as set forth in SEQ ID NO: 20, VL CDR2 as set forth in SEQ ID NO: 38, VL CDR3 as set forth in SEQ ID NO: 56;
(2) the following 3 heavy chain CDRs: VH CDR1 as set forth in SEQ ID NO: 21, VH CDR2 as set forth in SEQ ID NO: 39, VH CDR3 as set forth in SEQ ID NO: 57; and/or, the following 3 light chain CDRs: VL CDR1 as set forth in SEQ ID NO: 22, VL CDR2 as set forth in SEQ ID NO: 40, VL CDR3 as set forth in SEQ ID NO: 58;
(3) the following 3 heavy chain CDRs: VH CDR1 as set forth in SEQ ID NO: 23, VH CDR2 as set forth in SEQ ID NO: 41, VH CDR3 as set forth in SEQ ID NO: 59; and/or, the following 3 light chain CDRs: VL CDR1 as set forth in SEQ ID NO: 24, VL CDR2 as set forth in SEQ ID NO: 42, VL CDR3 as set forth in SEQ ID NO: 60;
(4) the following 3 heavy chain CDRs: VH CDR1 as set forth in SEQ ID NO: 25, VH CDR2 as set forth in SEQ ID NO: 43, VH CDR3 as set forth in SEQ ID NO: 61; and/or, the following 3 light chain CDRs: VL CDR1 as set forth in SEQ ID NO: 26, VL CDR2 as set forth in SEQ ID NO: 44, VL CDR3 as set forth in SEQ ID NO: 62;
(5) the following 3 heavy chain CDRs: VH CDR1 as set forth in SEQ ID NO: 27, VH CDR2 as set forth in SEQ ID NO: 45, VH CDR3 as set forth in SEQ ID NO: 63; and/or, the following 3 light chain CDRs: VL CDR1 as set forth in SEQ ID NO: 28, VL CDR2 as set forth in SEQ ID NO: 46, VL CDR3 as set forth in SEQ ID NO: 64;
(6) the following 3 heavy chain CDRs: VH CDR1 as set forth in SEQ ID NO: 29, VH CDR2 as set forth in SEQ ID NO: 47, VH CDR3 as set forth in SEQ ID NO: 65; and/or, the following 3 light chain CDRs: VL CDR1 as set forth in SEQ ID NO: 30, VL CDR2 as set forth in SEQ ID NO: 48, VL CDR3 as set forth in SEQ ID NO: 66;
(7) the following 3 heavy chain CDRs: VH CDR1 as set forth in SEQ ID NO: 31, VH CDR2 as set forth in SEQ ID NO: 49, VH CDR3 as set forth in SEQ ID NO: 67; and/or, the following 3 light chain CDRs: VL CDR1 as set forth in SEQ ID NO: 32, VL CDR2 as set forth in SEQ ID NO: 50, VL CDR3 as set forth in SEQ ID NO: 68;
(8) the following 3 heavy chain CDRs: VH CDR1 as set forth in SEQ ID NO: 33, VH CDR2 as set forth in SEQ ID NO: 51, VH CDR3 as set forth in SEQ ID NO: 69; and/or, the following 3 light chain CDRs: VL CDR1 as set forth in SEQ ID NO: 34, VL CDR2 as set forth in SEQ ID NO: 52, VL CDR3 as set forth in SEQ ID NO: 70; or
(9) the following 3 heavy chain CDRs: VH CDR1 as set forth in SEQ ID NO: 35, VH CDR2 as set forth in SEQ ID NO: 53, VH CDR3 as set forth in SEQ ID NO: 71; and/or, the following 3 light chain CDRs: VL CDR1 as set forth in SEQ ID NO: 36, VL CDR2 as set forth in SEQ ID NO: 54, VL CDR3 as set forth in SEQ ID NO: 72;
preferably, the antibody or antigen-binding fragment thereof further comprises a framework region of a human immunoglobulin.

3. The antibody or antigen-binding fragment thereof according to claim 1 or 2, wherein the antibody or antigen-binding fragment thereof comprises:
(a) a heavy chain variable region (VH) comprising an amino acid sequence selected from the following:
(i) the sequence as set forth in any one of SEQ ID NOs: 1, 3, 5, 7, 9, 11, 13, 15, or 17;
(ii) a sequence having a substitution, deletion or addition of one or several amino acids (e.g., a substitution, deletion or addition of 1, 2, 3, 4, or 5 amino acids) as compared to the sequence as set forth in any one of SEQ ID NOs: 1, 3, 5, 7, 9, 11, 13, 15, or 17; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 1, 3, 5, 7, 9, 11, 13, 15, or 17;
and/or
(b) a light chain variable region (VL) comprising an amino acid sequence selected from the group consisting of:
(iv) the sequence as set forth in SEQ ID NO: 2, 4, 6, 8, 10, 12, 14, 16 or 18;
(v) a sequence having a substitution, deletion or addition of one or several amino acids (e.g., a substitution, deletion or addition of 1, 2, 3, 4, or 5 amino acids) as compared to a sequence as set forth in SEQ ID NO: 2, 4, 6, 8, 10, 12, 14, 16 or 18; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2, 4, 6, 8, 10, 12, 14, 16 or 18;
preferably, the substitution described in (ii) or (v) is a conservative substitution;
preferably, the antibody or antigen-binding fragment thereof comprises:
(1) a VH having a sequence as set forth in SEQ ID NO: 1 and a VL having a sequence as set forth in SEQ ID NO: 2;
(2) a VH having a sequence as set forth in SEQ ID NO: 3 and a VL having a sequence as set forth in SEQ ID NO: 4;
(3) a VH having a sequence as set forth in SEQ ID NO: 5 and a VL having a sequence as set forth in SEQ ID NO: 6;
(4) a VH having a sequence as set forth in SEQ ID NO: 7 and a VL having a sequence as set forth in SEQ ID NO: 8;
(5) a VH having a sequence as set forth in SEQ ID NO: 9 and a VL having a sequence as set forth in SEQ ID NO: 10;
(6) a VH having a sequence as set forth in SEQ ID NO: 11 and a VL having a sequence as set forth in SEQ ID NO: 12;
(7) a VH having a sequence as set forth in SEQ ID NO: 13 and a VL having a sequence as set forth in SEQ ID NO: 14;
(8) a VH having a sequence as set forth in SEQ ID NO: 15 and a VL having a sequence as set forth in SEQ ID NO: 16; or
(9) a VH having a sequence as set forth in SEQ ID NO: 17 and a VL having a sequence as set forth in SEQ ID NO: 18.

4. The antibody or antigen-binding fragment thereof according to any one of claims 1 to 3, wherein the antibody or antigen-binding fragment thereof comprises:
(a) a heavy chain constant region (CH) of a human immunoglobulin or variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived; and/or
(b) a light chain constant region (CL) of a human immunoglobulin or variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived;
preferably, the heavy chain constant region is an IgG heavy chain constant region, such as an IgG1, IgG2, IgG3 or IgG4 heavy chain constant region;
preferably, the light chain constant region is a κ light chain constant region or a λ light chain constant region;
preferably, the heavy chain constant region has a sequence as set forth in SEQ ID NO: 121;
preferably, the light chain constant region has a sequence as set forth in SEQ ID NO: 122.

5. The antibody or antigen-binding fragment thereof according to any one of claims 1 to 4, wherein the antigen-binding fragment is selected from the group consisting of Fab, Fab', (Fab')², Fv, disulfide-linked Fv, scFv, diabody and single-domain antibody (sdAb); and/or the antibody is a murine antibody, a chimeric antibody, a humanized antibody, or a multispecific antibody;
preferably, the antigen-binding fragment is an Fab;
preferably, the antigen-binding fragment further comprises an Fc fragment (e.g., an Fc fragment of human IgG1) or mutant thereof;
preferably, the Fc fragment has a LALA mutation and a knob mutation; or, the Fc fragment has a LALA mutation and a hole mutation;
preferably, the Fc fragment has a sequence as set forth in SEQ ID NO: 111 or 112.

6. An isolated nucleic acid molecule, which encodes the antibody or antigen-binding fragment thereof according to any one of claims 1 to 5.

7. A vector, which comprises the nucleic acid molecule according to claim 6; preferably, the vector is a cloning vector or an expression vector.

8. A host cell, which comprises the nucleic acid molecule according to claim 6 or the vector according to claim 7; preferably, the host cell is a mammalian cell.

9. A method for preparing the antibody or antigen-binding fragment thereof according to any one of claims 1 to 5, comprising culturing the host cell according to claim 8 under a condition that allows expression of the antibody or antigen-binding fragment thereof, and recovering the antibody or antigen-binding fragment thereof from a culture of the cultured host cell.

10. A multispecific molecule, which comprises the antibody or antigen-binding fragment thereof according to any one of claims 1 to 5;
preferably, the multispecific molecule is capable of specifically binding to c-Met and additionally specifically binding to one or more other targets;
preferably, the multispecific molecule is a bispecific molecule;
preferably, the bispecific molecule further comprises a molecule (e.g., a second antibody) having a second binding specificity for a second target.

11. An immunoconjugate, which comprises the antibody or antigen-binding fragment thereof according to any one of claims 1 to 5 or the multispecific molecule according to claim 10, and a therapeutic agent linked to the antibody or antigen-binding fragment thereof or the multispecific molecule;
preferably, the therapeutic agent is selected from cytotoxic agents;
preferably, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof;
preferably, the immunoconjugate is an antibody-drug conjugate (ADC).

12. A pharmaceutical composition, which comprises the antibody or antigen-binding fragment thereof according to any one of claims 1 to 5, or the multispecific molecule according to claim 10, or the immunoconjugate according to claim 11, and a pharmaceutically acceptable carrier and/or excipient;
preferably, the pharmaceutical composition further comprises an additional pharmaceutically active agent;
preferably, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus;
preferably, the antibody or antigen-binding fragment thereof, the multispecific molecule or the immunoconjugate and the additional pharmaceutically active agent are provided as separate components or as components of the same composition.

13. A kit, which comprises the antibody or antigen-binding fragment thereof according to any one of claims 1 to 5;
preferably, the antibody or antigen-binding fragment thereof comprises a detectable label, such as an enzyme (e.g., horseradish peroxidase), a radionuclide, a fluorescent dye, a luminescent substance (e.g., a chemiluminescent substance) or biotin;
preferably, the kit further comprises a second antibody capable of specifically recognizing the antibody or antigen-binding fragment thereof according to any one of claims 1 to 5;
preferably, the second antibody further comprises a detectable label, such as an enzyme (e.g., horseradish peroxidase), a radionuclide, a fluorescent dye, a luminescent substance (e.g., a chemiluminescent substance) or biotin.

14. A chimeric antigen receptor, which comprises an antigen-binding domain of the antibody or antigen-binding fragment thereof according to any one of claims 1 to 5;
preferably, the antigen-binding domain comprises a heavy chain variable region and a light chain variable region of the antibody or antigen-binding fragment thereof according to any one of claims 1 to 5;
preferably, the antigen-binding domain is a scFv;
preferably, the chimeric antigen receptor is expressed by an immune effector cell (e.g., a T cell).

15. A method for inhibiting the growth of a tumor cell expressing c-Met and/or killing the tumor cell, comprising contacting the tumor cell with an effective amount of the antibody or antigen-binding fragment thereof according to any one of claims 1 to 5, or the multispecific molecule according to claim 10, or the immunoconjugate according to claim 11, or the pharmaceutical composition according to claim 12, or the chimeric antigen receptor according to claim 14.

16. Use of the antibody or antigen-binding fragment thereof according to any one of claims 1 to 5, or the multispecific molecule according to claim 10, or the immunoconjugate according to claim 11, or the pharmaceutical composition according to claim 12, or the chimeric antigen receptor according to claim 14 in the manufacture of a medicament for preventing and/or treating a tumor in a subject (e.g., a human);
preferably, the medicament further comprises an additional pharmaceutically active agent; preferably, the additional pharmaceutically active agent is a drug having an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus;
preferably, the tumor expresses c-Met;
preferably, the tumor involves a tumor cell expressing c-Met; preferably, the c-Met is expressed on the surface of the tumor cell;
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer,
thymic cancer, leukemia, lymphoma, myeloma, mycosis fungoids, Merkel cell carcinoma and other malignant blood diseases, such as classical Hodgkin's lymphoma (CHL), primary mediastinal large B cell lymphoma, B cell rich lymphoma of T cell/histiocyte, EBV-positive and -negative PTLD and EBV-related diffuse large B cell lymphoma (DLBCL), plasmablastic lymphoma, extranodal NK/T cell lymphoma, nasopharyngeal carcinoma and HHV8-related primary effusion lymphoma, Hodgkin's lymphoma, central nervous system (CNS) tumors, such as primary CNS lymphoma, spinal axis tumor, brainstem glioma;
preferably, the subject is a mammal, such as a human.

17. Use of the antibody or antigen-binding fragment thereof according to any one of claims 1 to 5 in the manufacture of a kit, wherein the kit is used for determining whether a tumor can be treated by an anti-tumor therapy targeting c-Met;
(1) contacting a sample containing the tumor cell with the antibody or antigen-binding fragment thereof according to any one of claims 1 to 5;
(2) detecting the formation of a complex comprising the antibody or antigen-binding fragment thereof and c-Met;
preferably, the antibody or antigen-binding fragment thereof comprises a detectable label;
preferably, the c-Met is c-Met of a mammalian (e.g., a human, a monkey);
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell carcinoma and other hematological malignancies, such as classical Hodgkin's lymphoma (CHL), primary mediastinal large B-cell lymphoma, B-cell rich lymphoma of T-cell/histiocyte, EBV-positive and -negative PTLD and EBV-related diffuse large B-cell lymphoma (DLBCL), plasmablastic lymphoma, extranodal NK/T-cell lymphoma, nasopharyngeal carcinoma and HHV8-related primary effusion lymphoma, Hodgkin's lymphoma, central nervous system (CNS) tumors, such as primary CNS lymphoma, spinal axis tumor, brainstem glioma.
